# EUROPÄISCHE PATENTANMELDUNG

(11) **EP 1 481 959 A1**
(43) Veröffentlichungstag der Anmeldung: **01.12.2004**
(21) Anmeldenummer: 04010036.4
(22) Anmeldetag: 28.04.2004
(51) Int. Cl.: C07C 51/34, C07C 59/52, C07D 307/83

(54) **Verfahren zur Herstellung von Lactonen und von aromatischen Hydroxycarbonsäuren**

(30) Priorität: 22.05.2003 AT 7912003
(71) Anmelder: DSM Fine Chemicals Austria Nfg GmbH & Co KG, 4021 Linz (AT)
(72) Erfinder: Jary, Walther, 4853 Steinbach am Attersee (AT)
(74) Vertreter: Lindinger, Ingrid

(57) **Zusammenfassung**

Verfahren zur Herstellung von gegebenenfalls substituierten Hydroxyphenylessigsäuren der Formel 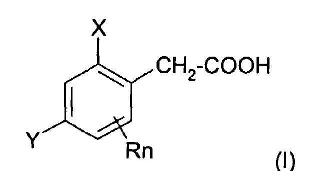 in der n 0, 1 2 oder 3 sein kann, R H oder einen C₁-C₆-Alkylrest, C₁-C₆-Alkoxyrest, Phenyl, Phenoxy, CN, NO₂, SO₃H, NH₂, F, Cl, I oder Br oder einen 5- oder 6-gliedrigen Ring mit einem N-Atom, der an den Phenylring anelliert ist bedeutet und die Reste X und Y OH, H, ein C₁-C₆-Alkylrest, ein C₁-C₆-Alkoxyrest, Phenyl, Phenoxy, CN, NO₂, SO₃H, NH₂, F, Cl, I oder Br sein können, wobei mindestens einer der Reste X oder Y OH bedeutet
oder von gegebenenfalls substituierten Lactonen der Formel 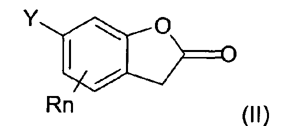 bei welchem ein Allylphenol der Formel 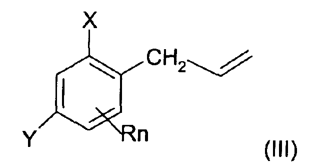 in Wasser, einem C₁-C₆-Alkohol oder einem Wasser/Alkoholgemisch mit Ozon korrespondierenden Aldehyd umgesetzt wird, der durch Erwärmen mit den bei der Ozonolyse entstehenden Peroxiden zur entsprechenden Hydroxyphenylessigsäure der Formel (I) aufoxidiert wird, die gegebenenfalls, für den Fall, dass X gleich OH ist, zum Lacton der Formel (II) cyclisiert werden kann.

## Beschreibung

Lactone, beispielsweise 2-Coumaron und aromatische Hydroxycarbonsäuren, wie etwa p-Hydroxyphenylessigsäure finden beispielsweise als Zwischenprodukte in der Herstellung von Agrochemikalien Anwendung.
Aus der Literatur sind bereits verschiedene Verfahren zur Herstellung von 2-Coumaron, das auch als 3H-2-Benzofuran-2-on bezeichnet wird, bekannt. Sie beruhen teils auf mehrstufiger Synthese und teils auf katalytischer Dehydrierung in der Gasphase.
R. W. Layer et al. (US 3 862 133) beschreiben die Synthese zur Herstellung von γ-Lactonen der o-Hydroxyphenylessigsäure, wobei der Aromat Substituenten trägt. Im Falle der Verwendung von Phenol und Glyoxal werden nur sehr geringe Ausbeuten an 2-Coumaron erhalten, da auf Grund der fehlenden Substituenten am Aromaten mehrere reaktive Positionen zu vielen Nebenprodukten führen.
Eine andere Methode zur Herstellung von 5-Chloro-3-H-benzofuran-2-on beschreiben J. C. Vallejos et al (FR 2 686 880), wobei von p-Chlorphenol und Glyoxylsäure in Anwesenheit von Phosphinsäure und katalytischen Mengen an Jod oder Salzsäure ausgegangen wird.
Andere Methoden zur Herstellung von 2-Coumaron sind zwar in der Literatur erwähnt, haben aber nie den Stellenwert einer industriellen Syntheseroute erreicht: T. Fukagawa et al. berichten über die intramolekulare Acyloxylation der Phenylessigsäure (J. Org. Chem., 1982, 47, 2491); E. Baciocchi et al. verwenden trans-2,3-Dichloro-2,3-dihydrobenzofuran als Edukt (J. Org. Chem., 1979, 44, 32) und H. E. Holmquist beschreibt eine Synthese ausgehend von o-Kresol und Kohlenmonoxid.
Unlängst beschrieben J. C. Vallejos et al. (US 5 616 733) ein Verfahren zur Herstellung von 2-Coumaron durch katalytische Dampfphasen-Dehydrierung des Kondensationsproduktes aus Cyclohexanon und wäßriger Glyoxylsäure, das vorerst in konzentrierter Essigsäure gelöst wird. Der größte Nachteile dieses Verfahrens liegt neben der raschen Katalysatordeaktivierung darin, dass nur sehr geringe Ausbeuten an 2-Coumaron erhalten werden.
Ebenfalls substituierte 3H-2-Benzofuran-2-one werden laut Beschreibung von Pfitzer Inc. (GB 1 337 507) ausgehend von 2-Methoxy-5-chlorphenylessigsäure hergestellt. Die Synthese der 2-Methoxy-5-chlorphenylessigsäure, anschließende Etherspaltung und Cyclisierung der 2-Hydroxy-5-chlorphenylessigsäure zu 2-Coumaron stellt kein kostengünstiges Verfahren dar.
2-Coumaron kann auch durch Dehydratisierung bzw. Cyclisierung von o-Hydroxyphenylessigsäure erhalten werden.
Zur Herstellung von o-Hydroxyphenylessigsäure, sowie von substituierten o-, m- oder p-Hydroxycarbonsäuren sind bereits ebenfalls eine Vielzahl von Herstellvarianten in der Literatur beschrieben.
Beispiele dafür sind die Herstellung von 2-Hydroxyphenylessigsäure durch Umwandlung von 2-Methoxyacetophenon in 2-Methoxyphenylessigsäure und Behandlung des Produktes mit Bromwasserstoffsäure (J. Org. Chem.11 (1946), S.798), ein Verfahren zur Herstellung von 2-Hydroxyphenylessigsäure durch Reduktion von Nitrophenylessigsäure und Diazotierung und Hydrolyse der resultierenden Aminophenylessigsäure (J. Chem. Soc. 1948, S. 150) ein Verfahren zur Herstellung von 2-Hydroxyphenylessigsäure durch Hydrolyse von 2-Bromphenylessigsäure in einer wässrigen Lösung von Morpholin in Gegenwart von Bis(ethylendiamin)-kupfer(II)-salz (J.O.C. 1971, S.2666-2668) und ein Verfahren zur Herstellung von 2-Hydroxyphenylessigsäure durch Umsetzung einer Halogenphenylessigsäure, wie 2-Chlorphenylessigsäure, mit einem Alkalimetallhydroxid in Gegenwart von metallischem Kupfer bei einer Temperatur von 220 bis 250°C und einem Druck von etwa 20 bis 60 bar (JP-OS-4870/1972) oder Verfahren zur Herstellung von Hydroxyphenylessigsäure durch durch Umsetzung einer Halogenphenylessigsäure mit einer Base in-Gegenwart eines Bis-(8-chinolinolat)-kupfer(II)-Komplexes (DE 3133583).
Die Nachteile dieser Verfahren liegen entweder in den extremen Reaktionsbedingungen, wie hohe Temperatur und/oder hoher Druck, oder in den schlechten Ausbeuten und/oder der schlechten Reinheit durch große Mengen an gebildeten Nebenprodukten oder bei der Verwendung von teuren und/ oder nicht umweltfreundlichen Edukten oder Reagenzien.

Aufgabe der vorliegenden Erfindung war es daher ein Verfahren zur Herstellung von gegebenenfalls substituierten Lactonen, sowie von gegebenenfalls substituierten o-oder p-Hydroxyphenylessigsäuren zu finden, bei welchem die Nachteile des Standes der Technik vermieden werden und das die gewünschten Edukte auf einfache und umweltfreundliche Weise in hoher Ausbeute und Reinheit liefert.

Unerwarteterweise konnte diese Aufgabe dadurch gelöst werden, dass als Edukt ein gegebenenfalls substituiertes Allylphenol verwendet wird, der durch Ozonolyse in die entsprechende Hydroxyphenylessigsäure umgesetzt wird, worauf gegebenenfalls die Cyclisierung zum entsprechenden Lacton erfolgen kann.

Gegenstand der vorliegenden Erfindung ist demnach ein Verfahren zur Herstellung von gegebenenfalls substituierten Hydroxyphenylessigsäuren der Formel 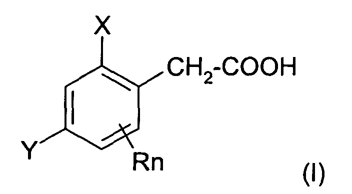 in der n 0, 1, 2 oder 3 sein kann, R H oder einen C₁-C₆-Alkylrest, C₁-C₆-Alkoxyrest, Phenyl, Phenoxyrest, CN, NH₂ , SO₃H, NO₂, F, Cl, I oder Br oder einen 5- oder 6-gliedrigen Ring mit einem N-Atom, der an den Phenylring anelliert ist bedeutet und die Reste X und Y OH, H, ein C₁-C₆-Alkylrest, ein C₁-C₆-Alkoxyrest, Phenylrest, Phenoxyrest, NH₂ , SO₃H, CN, NO₂, F, Cl, I Br sein können, wobei mindestens einer der Reste X oder Y OH bedeutet
oder von gegebenenfalls substituierten Lactonen der Formel 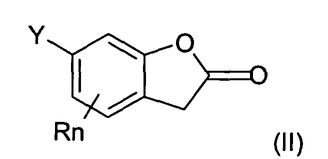 in der n, R und Y wie oben definiert sind, das dadurch gekennzeichnet ist, dass ein Allylphenol der Formel 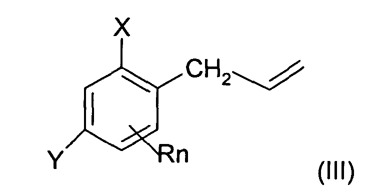 in der n, R, X und Y wie oben definiert sind, in Wasser, einem C₁-C₆-Alkohol oder einem Wasser/Alkoholgemisch mit Ozon zu dem korrespondierenden Aldehyd umgesetzt wird, der durch Erwärmen mit den bei der Ozonolyse entstehenden Peroxiden und bei Bedarf durch Zugabe von anderen Peroxiden zur entsprechenden Hydroxyphenylessigsäure der Formel (I) aufoxidiert wird, die gegebenenfalls, für den Fall, dass X gleich OH ist, zum Lacton der Formel (II) cyclisiert werden kann.

Gemäß der vorliegenden Erfindung werden Allylphenole der Formel (III) zuerst durch Ozonolyse und Oxidation der entstehenden Aldehyde in die korrespondierenden Hydroxyphenylcarbonsäuren der Formel (I) umgesetzt.

In den Formeln (I), (II) und (III) bedeutet n 0,1,2 oder 3 und R H oder einen C₁-C₆-Alkylrest, C₁-C₆-Alkoxyrest, Phenylrest, Phenoxyrest, SO₃H, CN, NO₂, NH₂ F, Cl, I oder Br oder einen 5- oder 6-gliedrigen Ring mit einem N-Atom, der an den Phenylring anniliert ist.
Die Reste X oder Y in den Formel (I), (II) und (III) bedeuten OH, H, einen C₁-C₆-Alkylrest, einen C₁-C₆-Alkoxyrest, Phenylrest, Phenoxyrest, NH₂, SO₃H, CN, NO₂, F, Cl, I oder Br, wobei mindestens einer der beiden Reste OH ist.

Unter C₁-C₆-Alkyl sind dabei lineare oder verzweigte Alkylreste, wie etwa Methyl, Ethyl, n-Propyl, i-Propyl, n-Butyl, tert.-Butyl, n-Pentyl, n-Hexyl u.s.w. zu verstehen.
C₁-C₆-Alkoxyreste sind beispielsweise Methoxy, Ethoxy, n-Propoxy, i-Popoxy, n-Butoxy, n-Hexoxy, u.s.w.
R kann aber auch ein an den Phenylring anellierten 5- oder 6-gliedrigen Ring mit einem N-Atom sein, sodass beispielsweise ein Chinolinring, ein Isochinolinring, ein Indol- oder ein Isoindolring erhalten wird.
Bevorzugt bedeutet R jedoch einen C₁-C₄-Alkyl- oder Alkoxyrest, besonders bevorzugt einen C₁-C₂-Alkyl- oder Alkoxyrest.

Als Edukt dient ein Allylphenol der Formel (III), in der n und R obige Bedeutung haben.
Die OH-Gruppe kann dabei in o- oder p-Stellung zur Allylgruppe stehen.
Allylphenole der Formel (III) sind, wie etwa 2-Allylphenol, zum Teil käuflich erwerbbar oder können durch bekannte Methoden, beispielsweise gemäß DE 27 46 002 aus Phenol und Allylchlorid oder gemäß US 3,526,668 aus substituierten Phenolen mit einem Allylhalogenid, hergestellt werden.
Bevorzugt werden die eingesetzten Allylphenole der Formel (III) aus einem gegebenenfalls substituierten Phenol der Formel 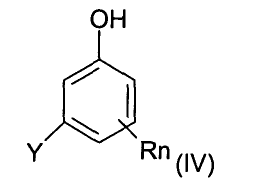 in der n, R und Y wie oben definiert sind, hergestellt.
Dabei wird das gegebenenfalls substituierte Phenol in einer inertisierten Apparatur vorgelegt und in einem geeigneten Lösungsmittel aus der Gruppe Cyclohexanon, Methyl-i-butylketon, Methanol, Ethanol, Aceton oder 2-Butanon gelöst. Anschließend werden ein geeigneter Katalysator, wie etwa Kl, Nal, u.s.w. und eine Base, wie etwa Kaliumcarbonat, Natriummethylat, Natriumhydroxyd, u.s.w. zugesetzt und das Reaktionsgemisch auf 30°C bis 130°C erhitzt, worauf die Zugabe von Allylchlorid in einer Zeit von 5 min bis zu 5 Stunden erfolgt. Allylchlorid wird dabei in äquimolarer Menge oder im molaren Überschuss von 1 mol bis 2mol pro mol Phenol zugesetzt. Es wird sodann für einige Zeit bei dieser Temperatur weitergerührt.
Der so erhaltene Allylphenylether wird sodann aus dem Reaktionsgemisch isoliert. Dazu wird zuerst das Lösungsmittel abdestilliert und ev. überschüssiges Phenol durch Wäsche des Rohproduktes mit NaOH abgetrennt, worauf durch Zugabe von Methyl-tert.-Butylether ein Phasentrennung erfolgt. Die organische Phase wird sodann gegebenenfalls noch durch eine schwach saure Wäsche, beispielsweise mit schwach sauren Phosphatpuffern, von Resten der NaOH gereinigt, getrocknet, filtriert und vom Lösungsmittel befreit.

Anschließend erfolgt die Umlagerung des gegebenenfalls substituierten Allylphenylethers zum korrespondierenden Allylphenol der Formel (III). Die Umlagerung erfolgt dabei durch Erhitzen auf eine Temperatur von 180 -250°C, bevorzugt unter Stickstoffatmosphäre.
Die Umlagerung kann dabei ohne Lösungsmittel oder in einem unter den Reaktionsbedingungen inerten Lösungsmittel, wie etwa Xylol, durchgeführt werden.

Je nach Stellung der Substituenten R und der OH-Gruppe werden dabei o- oder p-Allylphenole erhalten.
So lagert sich beispielsweise ein unsubstituierter Allylphenylether in das entsprechende o-Allylphenol um. Befindet sich in Position 2 ein Substituent, so lagert sich der Allylrest an die Position 6 des Phenylringes an. Sind sowohl Position 2 und 6 substituiert, so erfolgt die Umlagerung des Allylrestes zur Position 4, sodass das entsprechende disubstituierte p-Allylphenol erhalten wird.

Bei dem erfindungsgemäßen Verfahren wird das gegebenenfalls substituierte Allylphenol mit Ozon zum korrespondierenden Aldehyd umgesetzt.

Die Umsetzung erfolgt dabei in Wasser, einem C₁-C₆-Alkohol oder einem Wasser/Alkoholgemisch.
Als C₁-C₆-Alkohol eignen sich Methanol, Ethanol, n-Propanol, i-Propanol, n-Butanol, Pentanol oder Hexanol. Bevorzugt werden C₁-C₄-Alkohole eingesetzt. Besonders bevorzugt wird als Alkohol Methanol verwendet.
Erfolgt die Ozonolyse in Wasser oder in einem Wasser/Alkohol-Gemisch mit hohem Wasseranteil, so wird dem Reaktionsgemisch gegebenenfalls ein Entschäumer zugesetzt.
Ozon wird dabei in äquimolarer Menge, bezogen auf das Allylphenol zugesetzt. Gegebenenfalls kann auch ein geringer Überschuss an Ozon verwendet werden.

Die Reaktionstemperatur liegt dabei in Abhängigkeit vom verwendeten Lösungsmittel bei -30°C bis +30°C, bevorzugt bei -20°C bis+20°C.

Nach beendeter Ozonolyse wird der so erhaltene Aldehyd sogleich in einem Eintopfverfahren durch Erwärmen mit den bei der Ozonolyse entstehenden Peroxiden zur entsprechenden Hydroxyphenylessigsäure der Formel (I) aufoxidiert.
Zur Vervollständigung der Oxidation kann gegebenenfalls ein anderes Peroxid, wie etwa H₂O₂, zugegeben werden.
Dazu wird das Reaktionsgemisch für 30min bis zu 10h, bevorzugt für 1 h bis zu 6h, auf 40 bis 80°C, bevorzugt auf 45 bis 65°C erhitzt.
Nach beendeter Reaktion - die Reaktionskontrolle kann beispielsweise mittels Dünnschichtchromatographie erfolgen - wird das Reaktionsgemisch mit einem geeigneten Extraktionsmittel extrahiert.
Als Extraktionsmittel eignen sich Essigester, Dichlormethan, Toluol, u.s.w..
Die organische Phase wird sodann gegebenenfalls getrocknet und anschließend eingedampft, worauf die entsprechenden Hydroxyphenylessigsäure der Formel (I) erhalten wird.

Für den Fall, dass sich die OH-Gruppe in o-Stellung zum Essigsäurerest befindet (X gleich OH) kann die Hydroxyphenylessigsäure zum korrespondierenden Lacton cyclisiert werden.
Die Cyclisierung erfolgt dabei spontan beim Erhitzen oder durch Wasserabtrennung in einem Lösungsmittel, das das Entfernen von Wasser über einen Wasserabscheider erlaubt, wie etwa Toluol.
Bevorzugt werden unsubstituierte oder in Position 3, 4, 5 oder 6 substituierte o-Hydroxyphenylessigsäuren cyclisiert, wodurch gegebenenfalls substituierte 2-Coumarone erhalten werden.

Durch das erfindungsgemäße Verfahren werden gegebenenfalls substituierte Hydroxycarbonsäuren und deren Lactone in einfacher Weise und in hohen Ausbeuten und Reinheiten erhalten.

### Beispiel 1: Herstellung des Allylethers

15g Phenol wurden in einer trockenen mit N₂ gespülten Apparatur, bestehend aus einem 3-Halskolben mit Rückflusskühler, Thermometer und Tropftrichter, vorgelegt, in 15ml Cyclohexanon gelöst und anschließend mit 2g Kaliumiodid und 24,3g Kaliumcarbonat versetzt.
Das Reaktionsgemisch wurde sodann auf 80 - 90°C erhitzt und innerhalb von 3,5h mit 13,42g Allylchlorid versetzt, worauf anschließend bei 100°C gerührt wurde. Nach 5h betrug das Verhältnis von Allylether : Phenol 77:14.

### Beispiel 2: Herstellung des Allylethers

Beispiel 2 wurde analog Beispiel 1 durchgeführt, wobei anstelle von Cyclohexanon Methyl-tert.Butylketon als Lösungsmittel eingesetzt wurde und Allylchlorid innerhalb von 2,5h bei Siedetemperatur (120-125°C) zugetropft wurde. Nach 24h Erhitzen auf Siedetemperatur betrug das Verhältnis von Allylether : Phenol 82:10.

### Beispiel 3: Herstellung des Allylethers

10g Phenol wurden wie in Beispiel 1 vorgelegt, in 35ml MeOH gelöst und innerhalb von 10min mit 18,2g 30%iger Natriummethylat-Lösung in MeOH versetzt, wobei die Temperatur anstieg. Gleichzeitig wurde das Reaktionsgemisch erhitzt, sodass bei Ende der Zugabe 65°C (Siedetemperatur) erreicht wurde. Nach 15min wurden 1,59g Natriumiodid zugegeben, welches sich vollständig löste. Danach wurden 9,8g Allylchlorid innerhalb von 10 Minuten zugesetzt., wobei NaCl ausfiel.
Nach 4h Reaktionszeit wurde das Lösungsmittel abdestilliert, der Rückstand auf Raumtemperatur abgekühlt und mit 10%iger NaOH versetzt. Der Niederschlag löste sich und nach Zugabe von MTBE konnten die Phasen getrennt werden. Die wässrige Phase wurde noch 2mal mit MTBE extrahiert, die vereinten organischen Phasen schwach sauer gewaschen, über Natriumsulfat getrocknet und filtriert. Nach Abziehen des Lösungsmittels konnten 12,71g (94% d.Th.) Allylphenylether in einer Reinheit von 94Fl% (GC) erhalten werden.

### Beispiel 4: Herstellung von 2-Allylphenol

Die Umlagerung des gemäß Beispiel 3 hergestellten Allylphenylethers erfolgte unter Stickstoffatmosphäre durch Erhitzen auf Siedetemperatur (192-210°C). Nach 6 Stunden war der Allylphenylether vollständig umgesetzt. Der Anteil an 2-Allylphenol betrug 85% (GC-FI%)

### Beispiel 5:

2 g [18,5 mmol] o-Kresol wurden in 100 ml THF aufgenommen und mit 3,4 ml [337 mmol] 44%-iger Natronlauge bei Raumtemperatur versetzt. Die erhaltene Suspension wurde bei guter Rührung mit 4,7 g [40 mmol] Allylbromid versetzt und bei Raumtemperatur für 30 Minuten gerührt. Die Reaktionslösung wurde auf 20 ml Wasser gegossen und mit 100 ml Dichlormethan drei mal extrahiert. Die vereinigten organischen Extrakte wurden über Natriumsulfat getrocknet und das Lösungsmittel wurde danach abdestilliert.
Rohausbeute: 3,25 g 119 % d.Th
Überschüssiges Allylbromid wurde im Vakuum abdestilliert.
Ausbeute: 2,4 g 88 % d.Th

### Beispiel 6:

Die Herstellung der Allylether erfolgte analog zu Beispiel 5 anstatt von o-Kresol wurden, m-Kresol, p-Methoxyphenol und m-Methoxyphenol eingesetzt.

Eingesetzte Mengen:

| Stoff | | Menge | mol |
|---|---|---|---|
| m-Kresol | | 2,0 g | 0,0185 |
| | NaOH 44% | 3,4 ml | 0,037 |
| | Allylbromid | 4,9 g | 0,041 |
| m-Methoxyphenol | | 2,0 | 0,0161 |
| | NaOH 44% | 2,0 g | 0,016 |
| | Allylbromid | 4,3g | 0,036 |
| p-Methoxyphenol | | 2,0 | 0,0161 |
| | NaOH | 3,0 ml | 0,032 |
| | Allylbromid | 4,3 g | 0,036 |

Rohausbeuten:
m-Kresolallylether: 3,35g
m-Methoxy-allyl-phenol: 3,74g
p-Methoxy-allyl-phenol: 3,54 g

Überschüssiges Allylbromid wurde mittels Destillation entfernt.

Ausbeuten:
m-Kresol-allylether: 2,7 g (99% d.Th.)
m-Methoxy-allyl-phenol: 2,6 g (97% d. Th)
p-Methoxy-allyl-phenol: 2,6 g (96 % d.Th)

### Beispiel 7:

In 200 ml Ethanol (wasserfrei) wurden 51,4 g (0,76 mol) Natriumethylat vorgelegt und bei Raumtemperatur 40 g (0,37 mol) m-Kresol zugegeben. Nach 10 Minuten wurden 41 ml (0,59 mol) Allylbromid zugetropft und 30 Minuten lang gerührt. Danach wurden 100 ml Ethanol abdestilliert und die Reaktionslösung wurde mit 100 ml Wasser verdünnt. Es wurde mit Dichlormethan extrahiert und die organische Phase mit 5 % Natronlauge nachgewaschen um das verbleibende Kresol zu entfernen. Die organische Phase wurde über Magnesiumsulfat getrocknet und eingedampft.

### Beispiel 8: Ozonolyse von 2-Allylphenol in Wasser

In einer 100 ml Batchapparatur wurden in 250ml Wasser 2,7g (0,02mol) 2-Allylphenol aufgenommen und auf 15°C abgekühlt und mit Entschäumer versetzt. Danach wurden 0,96g (0,02mol) Ozon durch die Lösung durchgeleitet. Nach beendeter Ozonolyse wurde der Ozonstrom abgeschaltet und 15 Minuten lang mit Stickstoff inertisiert.
Anschließend wurde der Reaktionsansatz in einen 2-Halskolben mit Rückflusskühler überführt und auf 60°C erhitzt. Die Reaktion wurde mittels Dünnschichtchromatographie kontrolliert. Nach beendeter Reaktion wurde bei 50°C mit 3 mal mit je 50ml Toluol extrahiert und die bereinigten Extrakte auf Rückflusstemperatur erhitzt und gleichzeitig das gebildete Wasser mittels Wasserabscheider abgetrennt.
Anschließend wurde das Toluol durch Eindampfen entfernt.
Es wurden 2,2g 2-Coumaron (85% d.Th.) erhalten

### Beispiel 9: Ozonolyse in Methanol/Wasser 1/10 v/v

In einer 100 ml Batchapparatur wurden in 275ml Methanol/Wasser (1/10 v/v) 2,7g (0,02mol) 2-Allylphenol aufgenommen und auf 5°C abgekühlt und mit Entschäumer versetzt. Danach wurden 0,96g (0,02mol) Ozon durch die Lösung durchgeleitet. Nach beendeter Ozonolyse wurde der Ozonstrom abgeschaltet und 15 Minuten lang mit Stickstoff inertisiert.
Anschließend wurde der Reaktionsansatz in einen 2-Halskolben mit Rückflusskühler überführt und auf 60°C erhitzt. Die Reaktion wurde mittels Dünnschichtchromatographie kontrolliert. Nach beendeter Reaktion (5h) wurde 2 mal mit je 100ml Essigester extrahiert und die vereinigten organischen Extrakte über Natriumsulfat getrocknet. Nach dem Eindampfen der Lösung erhielt man 2,8g Hydroxyphenylessigsäure (93% d.Th.)

## Patentansprüche

1. Verfahren zur Herstellung von gegebenenfalls von gegebenenfalls substituierten Hydroxyphenylessigsäuren der Formel 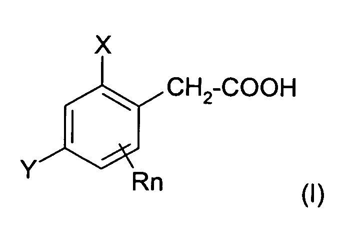 in der n 0, 1, 2 oder 3 sein kann, R H oder einen C₁-C₆-Alkylrest, C₁-C₆-Alkoxyrest, Phenylrest, Phenoxyrest, SO₃H, NH₂, CN, NO₂, F, Cl, I oder Br oder einen 5- oder 6-gliedrigen Ring mit einem N-Atom, der an den Phenylring anelliert ist, bedeutet und die Reste X und Y OH, H, ein C₁-C₆-Alkylrest, ein C₁-C₆-Alkoxyrest, Phenylrest, Phenoxyrest, CN, NO₂, SO₃H, NH₂, F, Cl, I oder Br sein können, wobei mindestens einer der Reste X oder Y OH bedeutet,
oder von gegebenenfalls substituierten Lactonen der Formel 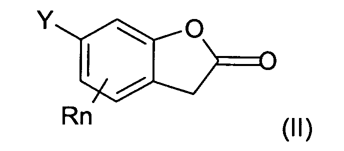 in der n, R und Y wie oben definiert sind, **dadurch gekennzeichnet, dass** ein Allylphenol der Formel 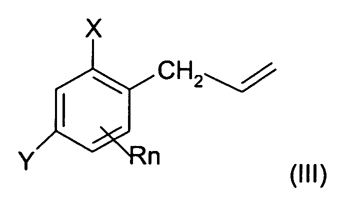 in der n, R, X und Y wie oben definiert sind, in Wasser, einem C₁-C₆-Alkohol oder einem Wasser/Alkoholgemisch mit Ozon zu dem korrespondierenden Aldehyd umgesetzt wird, der durch Erwärmen mit den bei der Ozonolyse entstehenden Peroxiden und bei Bedarf durch Zugabe von weiteren Peroxiden, zur entsprechenden Hydroxyphenylessigsäure der Formel (I) aufoxidiert wird, die gegebenenfalls, für den Fall, dass X gleich OH ist, zum Lacton der Formel (II) cyclisiert werden kann.

2. Verfahren nach Anspruch 1, **dadurch gekennzeichnet, dass** die Umsetzung mit Ozon bei einer Temperatur von -30°C bis +30°C erfolgt.

3. Verfahren nach Anspruch 1, **dadurch gekennzeichnet, dass** zur Vervollständigung der Oxidation zur Hydroxyphenylessigsäure der Formel (I) H₂O₂ zugegeben wird.

4. Verfahren nach Anspruch 1, **dadurch gekennzeichnet, dass** das Reaktionsgemisch zur Aufoxidation des Aldehyds zur Hydroxyphenylessigsäure der Formel (I) auf 40 bis 80°C erwärmt wird.

5. Verfahren nach Anspruch 1, **dadurch gekennzeichnet, dass**, falls X gleich OH ist, die Cyclisierung spontan beim Erwärmen oder durch Wasserabtrennung erfolgt.

6. Verfahren nach Anspruch 1, **dadurch gekennzeichnet, dass** die eingesetzten Allylphenole der Formel (III) aus den entsprechenden substituierten Phenolen der Formel (IV) 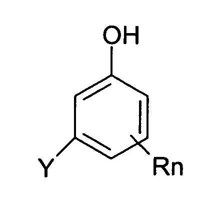 in der n, R und Y wie in der Formel (I) definiert sind, durch Umsetzung mit Allylchlorid in einem Lösungsmittel aus der Gruppe Cyclohexanon, Methyl-i-butylketon, Methanol, Ethanol, Aceton oder 2-Butanon in Gegenwart eines Katalysators und einer Base bei 30 bis 130°C und anschließender Umlagerung der so erhaltenen Allylphenylether bei 180°C bis 250°C hergestellt werden.
